# EUROPÄISCHE PATENTSCHRIFT

(11) **EP 1 567 260 B1**
(45) Veröffentlichungstag und Bekanntmachung des Hinweises auf die Patenterteilung: **31.12.2008**
(21) Anmeldenummer: 03758075.0
(22) Anmeldetag: 28.10.2003
(51) Int. Cl.: B01J 20/26, B01J 20/28, B01J 20/30, A61L 15/22, A61L 15/42

(54) **ULTRADÜNNE MATERIALIEN AUS FASER UND SUPERABSORBER**
ULTRA-THIN MATERIALS MADE FROM FIBRE AND SUPERABSORBENT
MATERIAUX ULTRAFINS CONSTITUES DE FIBRES ET D'UN POLYMERE SUPERABSORBANT

(30) Priorität: 31.10.2002 DE 10251137
(43) Veröffentlichungstag der Anmeldung: 31.08.2005
(73) Patentinhaber: BASF SE, 67056 Ludwigshafen (DE)
(72) Erfinder: HERMELING, Dieter, 67459 Böhl-Iggelheim (DE); BORDADO, Joao, Carlos, Moura, 1050-Lisboa (PT)
(86) Internationale Anmeldenummer: PCT/EP2003/011930
(87) Internationale Veröffentlichungsnummer: WO 2004/039493

(56) Entgegenhaltungen:
- EP-A- 0 666 350
- WO-A-01/18302
- WO-A-01/56625
- WO-A-95/30396
- WO-A-02/094329
- WO-A-03/053487

## Beschreibung

Die Erfindung betrifft Materialien aus superabsorbierendem Polymer (SAP) und Fasern, die durch Pressen bei Temperaturen von mindestens 60° C und Drucken von mindestens 3 bar erhältlich sind. Die Erfindung betrifft Materialien, die durch in situ Polymerisation aus SAP-Vorläufergemischen auf der Faser erhalten werden. Die Erfindung betrifft außerdem Verfahren zur Herstellung solcher Materialien und deren Verwendung.

In situ Materialien sind seit Anfang der 80er Jahre des letzten Jahrhunderts bekannt. Sie zeichnen sich dadurch aus das ein flächiges Fasergebilde, insbesondere ein sogenanntes non-woven mit flüssigem Medium behandelt wird, das nach der Polymerisation auf dem Fasergebilde (in-situ) ein absorbierendes Polymer bildet. Die Polymerisation kann durch alle bekannten Möglichkeiten ausgelöst werden, wie Strahlung (UV, Elektronenstrahl, Wärme), Zusätze (z.B. Redox-Starter). Das flüssige Medium enthält Monomere und eventuell Comonomere, die das absorbierende Polymer bilden. Vernetzer, weitere optionale Zusätze z.B. Geruchsinhibitoren, Verdicker, SAP Feinpulver etc können schon in dem flüssigem Medium vorhanden sein oder erst nach der Behandlung des Flächengebildes diesem zugegeben werden. Eine Nachbehandlung nach der Polymerisation, z.B. Oberflächennachvernetzung kann folgen. Die Behandlung des Flächengebildes mit dem flüssigem Medium kann durch Besprühen, tränken oder andere übliche Behandlungsmethoden geschehen.

In situ Materialien sind im Stand der Technik z.B. in EP 40 087, EP 54 841, EP 123 500, EP 108 637, EP 223 908, EP 315 185, WO 95/33878, WO 01/56625 bekannt.

Materialien aus SAP und Fasern, bei denen der SAP zu den Fasern gemischt wird, sind bekannt. Dies kann auf verschiedene Weise geschehen, z.B. durch Zugabe von SAP zum Prozess der Herstellung eines flächigen Fasermaterials (air laid oder wet laid) oder durch Zugabe des SAP, nachdem das Fasermaterial schon zu einem Flächengebilde geformt wurde. Der SAP kann dann durch verschiedene Methoden an den Fasern fixiert werden, z.B. durch Adhäsionsmittel. Der SAP kann als Schicht auch zwischen zwei Faserschichten eingelagert werden (siehe z.B.: WO 95/30396).

Für viele Anwendungen im Hygienebereich und Anwendungen außerhalb der Hygiene, bei denen wässrige Flüssigkeiten absorbiert werden sollen, wären Materialien wünschenswert, die eine, bevorzugt mehrere der folgenden Eigenschaften aufweisen: im Wesentlichen, bei Kontakt mit Flüssigkeit nur in eine Richtung expandieren, um Lager- und Transportkosten gering zu halten in komprimierter Form vorliegen und während der Lagerung die Form beibehalten, die eine hohe Saugfähigkeit für wässrige Lösungen haben, z.B. gemessen im Teebeuteltest, die schnell bei Flüssigkeitsaufnahme ohne Druck und unter Druck sind, die geeignet sind als Komponente von Laminaten zu dienen.

Überraschenderweise wurde gefunden, dass Material aus superabsorbierendem Polymer und Fasern, erhältlich durch in-situ Aufpolymerisation des superabsorbierenden Polymers auf die Fasern und Pressen bei einer Temperatur von mindestens 60° C und höchstens 200 °C bei einem Druck von mindestens 3 bar die gewünschten Eigenschaften aufweist.

Eine Komprimierung durch Einwirkung von Druck zur Herstellung "ultradünner" Hygieneartikel wird in der WO 01 / 56625 beschrieben. Allerdings wird das Material über einen Zeitraum von 48 Sekunden einem Druck von ca. 5,5 bar (Gewebefläche: 0,056 m²; 7.000 pounds load) und einer Temperatur von 50 °C unterworfen. Auf diese Weise wird eine Komprimierung von ursprünglich 4,5 mm auf 0,67 mm erreicht. Diese Versuchsbedingungen wurden reproduziert und es wurden zwei Unterschiede und Nachteile gegenüber der jetzigen Erfindung festgestellt:
a) das Material ist nicht dimensionsstabil, d.h. nach 2 Wochen findet eine Ausdehnung auf bis zu 1,5 mm und nach 8 Wochen auf bis zu 2,4 mm statt.
b) mit der in der vorliegenden Erfindung beschriebenen Methode lassen sich wesentlich dünnere, aber dennoch sehr flexible Materialien herstellen als das in der WO 01 / 56625 beschriebene Material.

Durch Einwirkung von Temperatur und Druck lassen sich Materialien aus SAP und Fasern, z.B. SAP-Nonwoven Composites, die nach WO 01 / 56625 hergestellt werden können, komprimieren.

Die Komprimierung erfolgt in der Dimension, in der die Druckeinwirkung ausgeübt wird. Die beiden anderen Dimensionen bleiben durch die Komprimierung nahezu unverändert.

Die Komprimierung kann in der Weise erfolgen, dass man das Material zunächst auf die erforderliche Temperatur erwärmt und anschließend Druck einwirken lässt; ebenso kann das Material zuerst einer Druckeinwirkung ausgesetzt werden und anschließend auf die erforderliche Temperatur erwärmt werden; bevorzugt wird das Material jedoch unter gleichzeitiger Einwirkung von Druck und Temperatur komprimiert.

Eine Komprimierung des Materials kann sowohl diskontinuierlich - z.B. mit Pressen - als auch kontinuierlich - z.B. mit Kalandern - durchgeführt werden.

Bei der vorliegenden Erfindung kann gezeigt werden, dass ein Weg um Materialien mit den gewünschten Eigenschaften zu erhalten eine Einwirkung von Druck und Temperatur ist. Dabei lassen sich die Materialeigenschaften durch Variation der Temperatur wesentlich stärker beeinflussen als durch eine Variation des Druckes. Es wurde auch festgestellt, dass bereits relativ geringe Drucke ab 3 bar, z.B. 3 bar, 3.5 bar, 4 bar oder 4.5 bar zur Herstellung der neuen Materialien ausreichen. Bevorzugt werden Drucke von 5 oder mehr bar, z.B. 5, 5.5, 6, 6.5, 7, 7.5, 8, 8.5, 9, 9.5 bar, insbesondere bevorzugt sind Drucke über 10 bar, z.B. 10, 11, 12, 13, 14, 15, 20, 25, 30, 25, 40, 45, 50 und mehr bar. Drucke von mehr als 100 bar führen dagegen in der Regel nicht zu einer weiteren Verbesserung der Materialeigenschaften. Temperatur im Sinne dieser Erfindung ist die Temperatur im zu pressenden bzw. gepressten Material. Bei längeren Verweilzeiten beim Pressvorgang (z.B. 1 Minute) wird die Temperatur im Material, der Temperatur auf der Oberfläche der Presse im wesentlichen entsprechen. Temperaturen unterhalb von 60° C sind in der Regel nicht ausreichend. Im allgemeinen werden Temperaturen von 60°C und mehr verwendet, so z.B. 60° oder 65°C. Bevorzugt sind Temperaturen von 70° C oder mehr, so z.B. 70° C, 75° C, insbesondere von 80° C oder mehr, wie z.B. 80° C, 85° C, 90° C, 95° C, 100° C oder mehr. Die maximale Temperatur ist abhängig von der Verweilzeit des Materials bei der Temperatur, da thermische Degradation des Materials vermieden werden sollte. Der optimale Temperaturbereich liegt zwischen 80 °C und 180 °C, dies können neben den oben aufgeführten Temperaturen z.B. solche bei 110° C, 120° C, 130° C, 140° C, 150° C, 160° C oder 170° C sein.. Bei Temperaturen oberhalb von 200 °C können sich die Eigenschaften verschlechtern.

Die gewünschten Materialeigenschaften lassen sich bereits nach sehr kurzen Zeiten einer Einwirkung von Druck und Temperatur erreichen. Verweilzeiten von 1 Minute sind in der Regel ausreichend. Längere Verweilzeiten schaden im Allgemeinen nicht, sind aber aus ökonomischen Gründen nicht wünschenswert. Typische Verweilzeiten liegen bei 10 Sekunden, 20 Sekunden, 30 Sekunden, 40 Sekunden, 50 Sekunden oder 60 Sekunden. Es ist aber auch möglich die Presszeit bei industrieller Fertigung kürzer zu gestalten, also z.B. Presszeiten von 1, 2, 3, 4, 5, 6, 7, 8, 9 oder 10 Sekunden zu verwenden. Bei kurzen Presszeiten werden die höheren Temperaturen verwendet werden. Die kurzen Verweilzeiten sind dafür ausschlaggebend, dass das komprimierte Material kontinuierlich z.B. mit Kalandern oder Walzenstühlen hergestellt werden kann.

Durch Einwirkung von Druck und Temperatur wird eine Komprimierung auf ≤ 20 %, bevorzugt ≤ 15 %, insbesondere ≤ 10 % der ursprünglichen Dicke erreicht.

Unter SAP wird in Zusammenhang mit dieser Erfindung superabsorbierendes Polymer verstanden. Superabsorbierendes Polymer zeichnet sich dadurch aus, dass es mindestens das 10fache seines Gewichts im CRC Test mit 0.9%iger NaCl Lösung aufnimmt. SAP ist aus dem Stand der Technik bekannt und ist in dieser Erfindung bevorzugt auf Polyacrylat Basis. Der SAP kann in jeder Form vorliegen z.B. teilchenförmig, als Faser, Film oder Schaum, teilchenförmig ist bevorzugt. Verschiedene SAPs und Ihre Herstellung sind z.B. in WO 01/56625 Seite 3 Zeile 37 bis Zeile 16 auf Seite 19 beschrieben.

Unter Fasern wird im Zusammenhang mit dieser Erfindung alle Fasern verstanden, die mit SAP im Stand der Technik kombiniert wurden. Bevorzugte Fasern sind solche, die als nonwoven vorliegen. Bevorzugte Fasern sind z.B. in WO 01/56625 Seite 19 Zeile 40 bis Zeile 27 auf Seite 20 beschrieben.

Unter Pressen wird die Einwirkung von Kraft auf die Oberfläche des Materials verstanden. Dies kann durch klassische Pressen, Kalander oder andere geeignete Mittel geschehen.

Mit der vorliegenden Erfindung wird erstmals Material zur Verfügung gestellt, das sich bei Wasserzugabe (destilliertes Wasser) oder Zugabe wässriger Flüssigkeiten (0.9%ige NaCl-Lösung) in einer Dimension um mindestens das 5-fache ausdehnt und sich in den zwei anderen Dimensionen um weniger wie 20% ausdehnt.

Durch Einwirkung von Wasser oder wässrigen Flüssigkeiten zeigt das Material ein nahezu eindimensionales Quellverhalten. In x- und y- Achse beträgt die Ausdehnung in Wasser in der Regel nicht mehr als 20%, bevorzugt nicht mehr wie 18%, 16%, 14%, oder 12%, besonders bevorzugt nicht mehr wie 10 %, 8%, 7%, oder 6%, insbesondere nicht mehr wie 5%, 4%, 3%, 2% oder gar 1 %, in der z-Achse quillt das Material auf mehr als das 5-fache, 6-fache, oder 7-fache, bevorzugt mehr wie das 8-fache, oder 9-fache, insbesondere mehr wie das 10-fache, 11-fache, 12-fache, 13-fache, 14-fache oder gar 15-fache oder mehr an.

Das Material ist nach Einwirkung von Druck und Temperatur dimensionsstabil, d.h. es findet keine bzw. nur eine geringfügige Ausdehnung in Richtung der Dimension statt, in der das Material komprimiert wurde.

Das erfindungsgemäße Material weist bevorzugt eine Zunahme der Dicke nach 60 Tagen nach Komprimierung von weniger wie 100%, bevorzugt weniger wie 80%, mehr bevorzugt weniger wie 60%, insbesondere weniger wie 50% bezogen auf die Dicke direkt nach der Komprimierung auf.

Das erfindungsgemäße Material ist bevorzugt erhältlich durch Druck und Temperaturbehandlung von Material erhältlich durch in-situ Polymerisation (wie oben definiert und aus dem Stand der Technik bekannt) des SAP.

Zur Herstellung der erfindungsgemäßen ultradünnen Materialien eignen sich insbesondere die in der WO 01/56625 aufgeführten Gewebe. Der SAP ist vorzugsweise "in-situ" auf dieses Gewebe aufpolymerisiert, es können aber beispielsweise auch Materialien erfindungsgemäß komprimiert werden, bei denen der SAP in das Gewebe eingestreut ist oder auf das Gewebe aufgeklebt ist.

Es ist möglich, dem Ausgangsmaterial vor der Komprimierung Additive zuzugeben. Dies kann beispielsweise durch Aufsprühen des Additivs oder einer Additivlösung, durch Tränken, sowie durch Einstreuen oder Aufkleben fester Additive erfolgen. Als Additive werden beispielsweise Riech- und Aromastoffe, Biozide und sonstige geruchshemmende Stoffe, sonstige Wirkstoffe, Düngemittel und Nährstoffe, Farbstoffe, Tenside, Salze, Polymere, Weichmacher u.a. bezeichnet.

Ferner ist es möglich, erfindungsgemäß Laminate (Mehrschichtmaterialien) herzustellen. Dabei sind sowohl "Sandwichstrukturen", d.h. zwischen einer Ober- und Unterschicht aus demselben Material befindet sich eine zweite, von Ober- und Unterschicht verschiedene Schicht als auch Strukturen mit zwei oder mehreren (teilweise) unterschiedlichen Schichten möglich.

Es lässt sich beispielsweise das Ausgangsmaterial zusammen mit Baumwollgewebe, Polyestergewebe, Baumwoll-Polyester Mischgewebe, Papier, Pappe durch einminütiges Komprimieren bei 150 °C und 80 bar in Laminate überführen, wobei das Ausgangsmaterial gleichzeitig komprimiert wird und den Zusammenhalt der Komponenten bewirkt.

Durch die Komprimierung wird erfindungsgemäß ein Material mit einer Dichte von mindestens 0.5 g/ccm, bevorzugt mindestens 0,6 g/ccm, mehr bevorzugt mindestens 0,7 g/ccm, besonders bevorzugt 0,8 g/ccm, ganz besonders bevorzugt mindestens 0,9 g/ccm, insbesondere bis mindestens 1 g/ccm und mehr erhalten. Die maximale Dichte ist im Allgemeinen nicht mehr wie 1,2 g/ccm. Vor dem Komprimieren beträgt die Dichte typischerweise etwa 0,05 g/ccm. Da das Material auch nach der Komprimierung flexibel ist, nimmt das komprimierte Material ein wesentlich geringeres Volumen ein als das Ausgangsprodukt.

Unter Dichte wird das Gewicht des Materials pro Volumeneinheit verstanden, wobei unter Volumen die Ausdehnung des Materials (Länge*Breite*Dicke) verstanden wird.

Das Verhältnis von Teebeutel zu Retention ist in 0,9 %iger NaCl Lösung üblicherweise größer 1,7 bevorzugt größer 1,9, mehr bevorzugt größer 2, insbesondere größer 2,2 (bei SAP Granulat liegt der Wert in der Größenordnung 1,2 bis 1,5). Dieser hohe Wert besagt, dass das komprimierte Material in der Lage ist, größere Mengen Wasser aufzunehmen als SAP Granulat. Dies könnte daran liegen, dass die höhere Wasseraufnahme durch die Poren in der sich entfaltenden Gewebestruktur (Schwammeffekt) entsteht. Nach der schnellen Wasseraufnahme kann das Wasser anschließend vom SAP aufgenommen werden (Wasserspeicherung). Damit ergibt sich mit dem komprimierten erfindungsgemäßen Material ein vergleichbares Eigenschaftsprofil wie mit Fluff plus SAP.

Das erfindungsgemäße Material weist bei der Retention in 0.9%iger NaCl-Lösung bevorzugt Werte größer 3 g/ccm, mehr bevorzugt größer 5 g/ccm, insbesonder größer 6.5 g/ccm oder gar 7 g/ccm auf.

Die Retention (auch in der Einheit g / g) ist mit dem gepressten Material höher als mit dem nicht komprimierten Ausgangsmaterial. Damit führt das Komprimieren zu einem Material mit besseren Eigenschaften als sie das Ausgangsmaterial besitzt. Die Retention wird um so höher, je höher die Temperatur für die Kompression gewählt wird.

Gibt man das komprimierte Material in eine Atmosphäre mit hoher Wasserdampfkonzentration, wird das Material weicher.

Das erfindungsgemäße Material weist bevorzugt eine FSEV nach 60 Sekunden auf, die im Vergleich zum unkomprimierten Material mindestens verdoppelt ist.

Das erfindungsgemäße Material weist außerdem bevorzugt eine FSEV nach 2 Minuten auf, die im Vergleich zum unkomprimierten Material mindestens 60% höher ist.

Das erfindungsgemäße Material weist bevorzugt eine EVUL nach 60 Sekunden auf, die im Vergleich zum unkomprimierten Material mindestens verdoppelt ist.

Das erfindungsgemäße Material weist außerdem bevorzugt eine EVUL nach 2 Minuten auf, die im Vergleich zum unkomprimierten Material mindestens 60% höher ist.

Das erfindungsgemäße Material weist bevorzugt eine AAP (0.7psi) in 0.9%iger NaCl-Lösung auf, die größer 5 g/ccm ist oder größer 6,5 g/ccm ist, bevorzugt größer 9 g/ccm, mehr bevorzugt größer 10 g/ccm, besonders bevorzugt größer 11 g/ccm, insbesondere größer 12 g/ccm oder gar größer 13 g/ccm ist.

Die erfindungsgemäßen Materialien eignen sich auch als (Mehrschicht) Material zur Absorption von Wasserdampf. Diese Eigenschaft ist reversibel und bleibt auch bei mehreren Absorption/Trocken-Zyklen erhalten.

Diese Materialien lassen sich unter anderem in folgenden Anwendungen zur Absorption von Wasser oder wässrigen Flüssigkeit, insbesondere Körperflüssigkeit, vorteilhaft verwenden: Hygieneartikel (z.B. als sogenannter Absorptioncore, Storage und/oder Aquisition Layer in Babywindeln oder Erwachseneninkontinenzartikeln, Damenbinden etc.), Bettunterlagen, OP-Tücher, Wundauflagen, Kompressen, Unterlagen für Tiertoiletten, Fußmatten, Matten zum Absorbieren von Schnee, Raumklimaverbesserung, Klimaverbesserung in Sitz- und Liegemöbeln, Schuhsohlen und -einlagen, Kleidungseinlagen, Kleidungsstücke, Tischtücher, Servietten, Reinigungstücher, Dichtungen oder Basismaterial für Dichtungen, z.B. im Bausektor, Rohrinnen- und - außendichtungen, Kabelummantelungen, Dämm- und Abdichtmaterial in der Bauindustrie, Dachbahnen (Wasser- und Dampfsperren), Dichtungsbahnen für Deponien, Hochwasserschutz (Gebäudeschutz, Tankanlagen), Wasserstraßen, Tunnelbauten, Straßenbau, Rohrauskleidung für Drilling, Trockenmittel für Transport und Lagerung (z.B. Getreide), Folien für Agro, incl. Erosionsschutz, Unterlagen für Pflanzen, Ummantelung von Wurzelballen, Matten zum Keimen von Saatgut, Matten für Zierpflanzen (Balkonkästen etc.), Dichtungsmaterial im Boden gegen aufsteigendes Wasser und Sickerwasser, Dekontamination von Böden (z.B. Entfernen von Schwermetallsalzen), Abfallbeutel, Verpackungen, Saugmatten für Transport feuchter und wasserabgebender Güter, Laminate, Filter, Feuerschutz.

Das komprimierte Material hat naturgemäß eine geringere Oberfläche als das Ausgangsmaterial und somit eine geringere Wasseraufnahmegeschwindigkeit. Die Absorptionsgeschwindigkeit lässt sich beispielsweise durch eine Vergrößerung der Oberfläche erhöhen. Geeignete Maßnahmen sind z.B. Aufrauhen der Oberfläche oder Komprimieren in Gegenwart von strukturgebenden, oberflächenvergrößernden Elementen.

### Prüfergebnisse

Als Basismaterial wurde Luquafleece IS von BASF Aktiengesellschaft eingesetzt. Es können üblicherweise Vliese mit einem Flächengewicht von 20 bis 2000 g/m² eingesetzt werden. In den folgenden Beispielen wurde ein PET Vlies mit einem Flächengewicht 100 g/m² verwendet (Sawafill 8135 der Firma Sandler). Luquafleece IS kann in Analogie zu Beispiel 9 von WO 01/56625 durch Beladung des o.g. Nonwovens mit 200 g/m² SAP (beidseitige Beschichtung mit je 100 g/m²) hergestellt werden. Es können auch andere Beladungen verwendet werden, die im allgemeinen zwischen 50 g/m² und 1000 g/m² liegen. Die Beladungen können von einer Seite oder beidseitig erfolgen. Bevorzugt sind Beladungen zwischen 100 g/m² und 300 g/m².

Als Vergleich wurde ein Material unter den in WO 01 / 56625 angegebenen Bedingungen komprimiert und getestet. Dieses Material wird mit "Vergleichsmaterial" bezeichnet.

Es wurden Proben, die bei 5, 10, 80 und 160 bar und 50, 100, 150 und 200 °C hergestellt wurden, jeweils in 0,9 %iger NaCl-Lösung sowie dest. Wasser vermessen. Folgende Aussagen können anhand der Prüfergebnisse gemacht werden:
Testmethoden, mit denen sich die erfindungsgemäßen Materialien am deutlichsten von Luquafleece und dem nach WO 01 / 56625 hergestellten komprimierten Material unterscheiden, sind CRC sowie Testmethoden mit der Maßeinheit "Gramm absorbierte Flüssigkeit / ccm".
Die Retention oder CRC wurde gemessen, wie in WO 01/56625 Seite 30 Zeile 40 ff beschrieben.
Die AUL oder AAP wurde gemessen, wie in WO 01/56625 Seite 30 Zeile 16 ff beschrieben. Der Teebeutel wurde bestimmt, wie die Retention nur ohne Zentrifugation.
Typische Werte für das erfindungsgemäße Material werden in folgender Übersicht angegeben, wobei in Klammern die Werte des nicht komprimierten Ausgangsmaterials und des gemäß WO 01/56625 bei geringeren Temperaturen komprimierten Materials angegeben sind.
   Retention (in 0,9%iger NaCl): 6 - 8,2 g/g (5,8 g/g & 5,9 g/g)
   Retention (in 0,9%iger NaCl): 1500 - 2200 g/m² (1495 g/m² & 1346 g/m²)
   Retention (in 0,9%iger NaCl): 4 - 7,5 g/ccm (0,3 g/ccm & 0,4 g/ccm)
   Teebeutel (in 0,9%iger NaCl): 10 - 20 g/ccm (0,8 g/ccm & 1,3 g/ccm)
   Dichte: 0,5 -1,2 g/ccm (0,05 g/ccm & 0,07 g/ccm)
   Ausdehnungsfaktor (in 0,9%iger NaCl): 10 - 21 (1,1 & 1,7)
   AAP (in 0,9%iger NaCl; 0,7psi): 10,5 -12,5 g/g (13 g/g & 12,3 g/g)
   AAP (in 0,9%iger NaCl; 0,7psi): 2500 - 3600 g/m² (3300 g/m² & 2968 g/m²)
   AAP (in 0,9%iger NaCl; 0,7psi): 6 - 14 g/ccm (0,6 g/g & 1,0 g/ccm)

   Retention (in dest. Wasser): 13,5 - 19 g/g (13,3 g/g & 12,9 g/g)
   Retention (in dest. Wasser): 4000 - 5400 g/m² (3455 g/m² & 3106 g/m²)
   Retention (in dest. Wasser): 10 - 16,5 g/ccm (0,7 g/ccm & 0,9 g/ccm)
   Teebeutel (in dest. Wasser): 15 - 33 g/ccm (1,3 g/ccm & 3,0 g/ccm)
   Ausdehnungsfaktor (in dest. Wasser): 15 - 33 (1,5 & 2,2)
   AAP (in dest. Wasser): 18 - 22,5 g/g (18,9 g/g & 19,5 g/g)
   AAP (in dest. Wasser; 0,7psi): 5000 - 6200 g/m² (4750 g/m² & 4735 g/m²)
   AAP (in dest. Wasser; 0,7psi): 10 - 22 g/ccm (0,9 g / g & 1,4 g/ccm)

Das Nonwoven ohne SAP zeigt grundsätzlich andere Eigenschaften als Luquafleece IS. Noch stärkere Unterschiede bestehen zwischen komprimiertem Nonwoven und komprimiertem Luquafleece IS. Das reine Nonwoven hat aufgrund des Schwammeffektes einen hohen Teebeutelwert von 37 g / g in NaCl und, weil bei diesem Effekt der Salzgehalt keine Rolle spielt, mit 38 g / g den gleichen Wert in dest. Wasser. Der CRC Wert ist aus gleichem Grund mit 0,4 g / g in beiden genannten Medien extrem niedrig; denn durch das Zentrifugieren wird die Flüssigkeit nahezu vollständig aus dem Gewebe entfernt.

Wird das Nonwoven durch Einwirkung von Druck und Temperatur komprimiert, quillt es in Gegenwart von Wasser nicht auf. Dementsprechend niedrig sind die Teebeutel- und CRC Werte. Ein Nonwoven das 1 Minute bei 200 °C und 80 bar gepresst wurde, zeigte in 0,9 %iger NaCl einen Teebeutelwert von 1,9 g / g und eine CRC von 0,3 g / g. Die Dicke des gepressten Materials blieb mit 0,1 mm unverändert. Diese Ergebnisse zeigen, dass eine Quellung in der z-Achse nur in Gegenwart von SAP möglich ist.

Ultradünne Materialien gemäß der vorliegenden Erfindung lassen sich prinzipiell auch herstellen, indem man SAP Granulat oder Pulver in ein Nonwoven hineinstreut, den SAP ggf. mit einem Kleber auf dem Nonwoven fixiert oder durch andere Techniken auf dem Gewebe verankert. Obwohl die physikalischen Eigenschaften ähnlich zu denen des gepressten in-situ Materials sind, haben Produkte, die reversibel auf dem Nonwoven fixiert sind den Nachteil, dass sich der SAP durch das Aufquellen in Gegenwart von Wasser wieder vom Nonwoven ablöst. Bei komprimierten Materialien, die "in-situ" auf Fasern aufpolymerisiert sind, ist der SAP hingegen fest auf der Faser verankert und löst sich auch in Gegenwart von Wasser nicht wieder ab.

### Dimensionsstabilität

Das komprimierte Material ist dimensionsstabil, d.h. auch nach längerer Lagerung bei Raumtemperatur und rel. Luftfeuchten von vorzugsweise weniger als 60 % dehnt sich das Material nicht oder nur unwesentlich aus. Diese Dimensionsstabilität wurde bei allen Proben festgestellt, die bei einer Temperatur von mehr als 60 °C und einem Druck von mehr als 5 bar komprimiert wurden. Bei dem nach WO 01 / 56625 hergestellten Vergleichsmaterial fand dagegen unter den o.g. Bedingungen eine Ausdehnung des Materials statt:

| Probe[mm] | Dicke direkt nach Komprimierung [mm] | Dicke nach 60 Tagen |
|---|---|---|
| 1 | 0,8 | 2,4 |
| 2 | 0,7 | 1,8 |
| 3 | 0,7 | 1,9 |
| 4 | 0,8 | 2,3 |

Ausdehnungsvolumen in Abhängigkeit von der Zeit

In WO 01 / 56625 wird diese Kenngröße beschrieben (Seite 31 Zeile 33 ff - FSEV). Mit einer SAP Beladung von 200 g/m² wurden in 0,9%iger NaCl folgende Werte angegeben:

| | |
|---|---|
| 5 Sekunden | 1,4 ml |
| 10 Sekunden | 2,2 ml |
| 30 Sekunden | 4,3 ml |
| 60 Sekunden | 5,9 ml |
| 120 Sekunden | 7,3 ml |
| 300 Sekunden | 8,5 ml |
| 600 Sekunden | 9,0 ml |

Mit komprimierten Materialien, die bei verschiedenen Drucken und Temperaturen (Dauer: 1 Minute) komprimiert wurden, wurden folgende Werte gemessen. Mit "Vergleich" ist die Probe bezeichnet, die nach der WO 01 / 56625 hergestellt wurde (5 bar, 48 s, 50 °C).

### Bestimmung des Ausdehnungsvolumens (FSEV)

| Zeit | 5bar 100°C | 10ba r 100°C | 80bar 100°C | 5bar 150°C | 10bar 150°C | 80bar 150°C | Vergleich |
|---|---|---|---|---|---|---|---|
| 10 Sek. | 2,4 ml | 2,4 ml | 2,2 ml | 1,3 ml | 2,2 ml | 0,1 ml | 2,4 ml |
| 30 Sek. | 6,8 ml | 7,1 ml | 7,5 ml | 5,7 ml | 6,4 ml | 0,7 ml | 5,8 ml |
| 60 Sek. | 8,7 ml | 9,3 ml | 10,1 ml | 9,0 ml | 9,3 ml | 4,2 ml | 7,5 ml |
| 120 Sek. | 9,8 ml | 10,5 ml | 11,1 ml | 10,7 ml | 10,8 ml | 10,1 ml | 8,7 ml |
| 300 Sek. | 10,4 ml | 11,3 ml | 11,8 ml | 11,5 ml | 11,6 ml | 11,5 ml | 9,5 ml |
| 600 Sek. | 10,6 ml | 11,5 ml | 12,0 ml | 11,7 ml | 11,8 ml | 11,8 ml | 9,9 ml |

Die Werte zeigen, dass die FSEV Werte des erfindungsgemäßen Materials (mit der Ausnahme 80 bar / 150 °C) bereits nach 30 -.60 Sekunden deutlich höher liegen als die des in WO 01 / 56625 beschriebenen komprimierten Materials. Die Werte zeigen ebenfalls, dass der Endwert nach etwa 300 Sekunden fast erreicht ist.

Führt man die Messung durch, indem man das Ausdehnungsvolumen unter einer Druckeinwirkung von 0,5 psi (EVUL, gemäß WO 01/56625, Seite 32, Zeile 6 ff) durchführt, ergibt sich ein ähnliches Bild:

### Bestimmung des Ausdehnungsvolumens (EVUL) bei 0,5 psi

| Zeit | 5bar 100°C | 10bar 100°C | 80bar 100°C | 5bar 150°C | 10bar 150°C | 80bar 150°C | Vergleich |
|---|---|---|---|---|---|---|---|
| 10 Sek. | 0,9 ml | 0,4 ml | 1,2 ml | 0,6 ml | 0,8 ml | 0,1 ml | 0,1 ml |
| 30 Sek. | 3,0 ml | 2,2 ml | 3,7 ml | 3,0 ml | 3,1 ml | 1,4 ml | 1,0 ml |
| 60 Sek. | 4,4 ml | 3,6 ml | 5,1 ml | 4,7 ml | 4,7 ml | 4,2 ml | 2,1 ml |
| 120 Sek. | 5,0 ml | 4,6 ml | 5,8 ml | 6,0 ml | 5,8 ml | 5,8 ml | 3,1 ml |
| 300 Sek. | 5,6 ml | 5,3 ml | 6,1 ml | 6,6 ml | 6,4 ml | 6,4 ml | 3,8 ml |
| 600 Sek. | 5,6 ml | 5,7 ml | 6,3 ml | 6,9 ml | 6,6 ml | 6,4 ml | 3,8 ml |

Bei einer Wasseraufnahme unter einem Druck von 0,5 psi sind die nach dem erfindungsgemäßen Verfahren hergestellten Proben schneller als die Vergleichsprobe. Lediglich die bei 80 °C / 150 bar hergestellte Probe ergibt nach 10 Sekunden den gleichen Wert, alle anderen Messergebnisse sind aber auch hier besser als bei der Vergleichsprobe.

### Aufnahme von Wasserdampf

Das komprimierte Material ist in der Lage, signifikante Mengen Wasserdampf zu absorbieren und bei niedrigen relativen Luftfeuchten wieder abzugeben (vergleichbare Eigenschaften wie Luquafleece IS). Verschiedene Probestücke (jeweils 100 x 100 mm), die eine Minute bei 160 bar und unterschiedlichen Temperaturen komprimiert wurden, wurden 24 h bei Raumtemperatur in einem Exsikkator bei 95 % rel. Luftfeuchte gelagert. Anschließend wurden die Proben an der Luft bei 23 °C und 45 % rel. Luftfeuchte getrocknet. Die Resultate sind der Tab. A zu entnehmen. Anschließend wurden die Proben erneut 24 h im Exsikkator bei 95 % rel. Feuchte gelagert und danach wiederum bei Raumtemperatur und 45 % rel. Feuchte getrocknet. Dieser Zyklus wurde ein drittes Mal durchgeführt. Die Ergebnisse nach dem dritten Zyklus sind der Tabelle B zu entnehmen.

**Tab. A**

| Probe | Feuchtigkeitsaufnahme Gewicht nach 24 h (in % bez. auf Startgewicht) | Feuchtigkeitsabnahme Gewicht nach 24 h (in % bez. auf Startgewicht) |
|---|---|---|
| Luquafleece IS | 143 | 107 |
| P1 (50°C/160bar) | 142 | 107 |
| P2 (100°C/160bar) | 144 | 107 |
| P3 (150°C/160bar) | 149 | 112 |
| P4 (200°C/160 bar) | 149 | 114 |

**Tab. B**

| Probe | Feuchtigkeitsaufnahme Gewicht nach 3 Zyklen (in % bez. auf Startgewicht) | Feuchtigkeitsabnahme Gewicht nach 3 Zyklen(in % bez. auf Startgewicht) |
|---|---|---|
| Luquafleece IS | 149 | 107 |
| P1 (50°C/160bar) | 152 | 107 |
| P2 (100°C/160bar) | 152 | 109 |
| P3 (150°C/160bar) | 157 | 113 |
| P4 (200°C/160bar) | 155 | 114 |

### Beispiele

Für die Prüfungen wurden Proben der Fläche 30 x 50 mm verwendet. Für die AAP Tests wurde ein kreisrundes Stück mit einem Durchmesser von 6 cm eingesetzt (28,3 cm² Fläche). Die Versuche wurden mit 0,7 psi durchgeführt.

Bei der Vermessung der 30 x 50 mm Proben wurde eine Quellung im wesentlichen in z-Richtung beobachtet. In x- oder y-Richtung wurde eine Quellung um ca. 10 % gemessen, die dritte Dimension blieb unverändert (in einer Achse ist das Gewebe leichter dehnbar als in der anderen Dimension).

Alle Tests wurden nach den gängigen Standardmethoden für SAP durchgeführt.

### Beispiel 1

Luquafleece IS wurde eine Minute bei 100 °C und 160 bar komprimiert. Es wurden 30 x 50 mm Proben vermessen. Dabei wurden folgende Daten in dest. Wasser erhalten:
Dicke des komprimierten Materials: 0,3 mm
CRC: 15,6 g / g
CRC: 4905 g / m²
Teebeutelwert: 30,6 g / ccm*)
Retention: 16,4 g / ccm*)
Dichte des eingesetzten Materials: 0,984 g / ccm
Ausdehnungsfaktor in z-Achse: 31,0
AAP (0,7 psi): 19,1 g / g
AAP (0,7 psi): 5689 g / m²
AAP (0,7 psi): 19,0 g / ccm
*) die Angabe bezieht sich auf das eingesetzte Material

### Beispiel 2

Luquafleece IS wurde eine Minute bei 100 °C und 80 bar komprimiert. Es wurden 30 x 50 mm Proben vermessen. Dabei wurden folgende Daten in dest. Wasser erhalten:
Dicke des komprimierten Materials: 0,3 mm
CRC: 15,7 g / g
CRC: 4592 g / m²
Teebeutelwert: 31,7 g / ccm*)
Retention: 15,3 g / ccm*)
Dichte des eingesetzten Materials: 1,042 g / ccm
Ausdehnungsfaktor in z-Achse: 28,7
AAP (0,7 psi): 19,8 g / g
AAP (0,7 psi): 5477 g / m²
AAP (0,7 psi): 18,2 g / ccm
*) die Angabe bezieht sich auf das eingesetzte Material

### Beispiel 3

Luquafleece IS wurde eine Minute bei 150 °C und 80 bar komprimiert. Es wurden 30 x 50 mm Proben vermessen. Dabei wurden folgende Daten in dest. Wasser erhalten:
Dicke des komprimierten Materials: 0,25 mm
CRC: 14,6 g / g
CRC: 3326 g / m²
Teebeutelwert: 32,9 g / ccm*)
Retention: 16,6 g / ccm*)
Dichte des eingesetzten Materials: 0,969 g / ccm
Ausdehnungsfaktor in z-Achse: 32,5
AAP (0,7 psi): 19,2 g / g
AAP (0,7 psi): 4382 g / m²
AAP (0,7 psi): 21,9 g / ccm
*) die Angabe bezieht sich auf das eingesetzte Material

### Beispiel 4

Luquafleece IS wurde eine Minute bei 200 °C und 80 bar komprimiert. Es wurden 30 x 50 mm Proben vermessen. Dabei wurden folgende Daten in dest. Wasser erhalten:
Dicke des komprimierten Materials: 0,25 mm
CRC: 18,4 g / g
CRC: 4577 g / m²
Teebeutelwert: 30,0 g / ccm*)
Retention: 15,3 g / ccm*)
Dichte des eingesetzten Materials: 0,959 g / ccm
Ausdehnungsfaktor in z-Achse: 28,0
AAP (0,7 psi): 22,4 g / g
AAP (0,7 psi): 6184 g / m²
AAP (0,7 psi): 20,6 g / ccm
*) die Angabe bezieht sich auf das eingesetzte Material

### Beispiel 5

Luquafleece IS wurde eine Minute bei 150 °C und 10 bar komprimiert. Es wurden 30 x 50 mm Proben vermessen. Dabei wurden folgende Daten in dest. Wasser erhalten:
Dicke des komprimierten Materials: 0,4 mm
CRC: 16,1 g / g
CRC: 4830 g / m²
Teebeutelwert: 24,5 g / ccm*)
Retention: 12,1 g / ccm*)
Dichte des eingesetzten Materials: 0,697 g / ccm
Ausdehnungsfaktor in z-Achse: 22,3
AAP (0,7 psi): 20,9 g / g
AAP (0,7 psi): 6042 g / m²
AAP (0,7 psi): 15,1 g / ccm
   *) die Angabe bezieht sich auf das eingesetzte Material
Vergleichsbeispiel 1 (Luquafleece IS)
Dicke des Luquafleece IS: 5,3 mm '
CRC: 13,3 g / g
CRC: 3455 g / m²
Teebeutelwert: 1,3 g / ccm*)
Retention: 0,73 g / ccm*)
Dichte des eingesetzten Materials: 0,051 g / ccm
Ausdehnungsfaktor in z-Achse: 1,5
AAP (0,7 psi): 18,9 g / g
AAP (0,7 psi): 4750 g / m²
AAP (0,7 psi): 0,9 g / ccm
*) die Angabe bezieht sich auf das eingesetzte Material

Vergleichsbeispiel 2 (Luquafleece IS 48 s bei 6 bar und 50 °C komprimiert)
Dicke des komprimierten Materials: 3,1 mm
CRC: 12,9 g / g
CRC: 3106 g / m²
Teebeutelwert: 2,0 g / ccm*)
Retention: 0,9 g / ccm*)
Dichte des eingesetzten Materials: 0,074 g / ccm
Ausdehnungsfaktor in z-Achse: 2,2
AAP (0,7 psi): 19,5 g / g
AAP (0,7 psi): 4735 g / m²
AAP (0,7 psi): 1,4 g / ccm
*) die Angabe bezieht sich auf das eingesetzte Material

Ein Erhöhen des Druckes bei niedrigen Temperaturen ergibt keine signifikante Verbesserung. Dies soll anhand des folgenden Beispiels belegt werden.
Vergleichsbeispiel 3 (Luquafleece IS 60 s bei 80 bar und 50 °C komprimiert)
Dicke des komprimierten Materials: 0,8 mm
Dicke des komprimierten Materials nach 4 Wochen: 1,2 mm
CRC: 12,5 g / g
CRC: 2730 g / m²
Teebeutelwert: 3,0 g / ccm*)
Retention: 1,9 g / ccm*)
Dichte des eingesetzten Materials: 0,159 g / ccm
Ausdehnungsfaktor in z-Achse: 4,6
AAP (0,7 psi): 18,4 g / g
AAP (0,7 psi): 4276 g / m²
AAP (0,7 psi): 3,1 g / ccm
*) die Angabe bezieht sich auf das eingesetzte Material

### Beispiel 6

Luquafleece IS wurde eine Minute bei 100 °C und 160 bar komprimiert. Es wurden 30 x 50 mm Proben vermessen. Dabei wurden folgende Daten in 0,9 %iger NaCl erhalten:
Dicke des komprimierten Materials: 0,3 mm
CRC: 6,5 g / g
CRC: 2069 g / m²
Teebeutelwert: 19,4 g / ccm*)
Retention: 6,9 g / ccm*)
Dichte des eingesetzten Materials: 0,977 g / ccm
Ausdehnungsfaktor in z-Achse: 19,0
AAP (0,7 psi): 10,6 g / g
AAP (0,7 psi): 3357 g / m²
AAP (0,7 psi): 11,2 g / ccm
*) die Angabe bezieht sich auf das eingesetzte Material

### Beispiel 7

Luquafleece IS wurde eine Minute bei 100 °C und 80 bar komprimiert. Es wurden 30 x 50 mm Proben vermessen. Dabei wurden folgende Daten in 0,9 %iger NaCl erhalten:
Dicke des komprimierten Materials: 0,3 mm
CRC: 7,3 g / g
CRC: 2151 g / m²
Teebeutelwert: 19,5 g / ccm*)
Retention: 7,2 g / ccm*)
Dichte des eingesetzten Materials: 1,073 g / ccm
Ausdehnungsfaktor in z-Achse: 19,3
AAP (0,7 psi): 11,3 g / g
AAP (0,7 psi): 3039 g / m²
AAP (0,7 psi): 10,1 g / ccm
*) die Angabe bezieht sich auf das eingesetzte Material

### Beispiel 8

Luquafleece IS wurde eine Minute bei 150 °C und 160 bar komprimiert. Es wurden 30 x 50 mm Proben vermessen. Dabei wurden folgende Daten in 0,9 %iger NaCl erhalten:
Dicke des komprimierten Materials: 0,25 mm
CRC: 6,4 g / g
CRC: 1489 g / m²
Teebeutelwert: 16,5 g / ccm*)
Retention: 7,4 g / ccm*)
Dichte des eingesetzten Materials: 1,113 g / ccm
Ausdehnungsfaktor in z-Achse: 20,0
AAP (0,7 psi): 11,2 g / g
AAP (0,7 psi): 2650 g / m²
AAP (0,7 psi): 13,3 g / ccm
*) die Angabe bezieht sich auf das eingesetzte Material

### Beispiel 9

Luquafleece IS wurde eine Minute bei 150 °C und 80 bar komprimiert. Es wurden 30 x 50 mm Proben vermessen. Dabei wurden folgende Daten in 0,9 %iger NaCl erhalten:
Dicke des komprimierten Materials: 0,25 mm
CRC: 6,5 g / g
CRC: 1490 g / m²
Teebeutelwert: 20,1 g / ccm*)
Retention: 7,5 g / ccm*)
Dichte des eingesetzten Materials: 1,006 g / ccm
Ausdehnungsfaktor in z-Achse: 20,5
AAP (0,7 psi): 11,7 g / g
AAP (0,7 psi): 2792 g / m²
AAP (0,7 psi): 14,0 g / ccm
*) die Angabe bezieht sich auf das eingesetzte Material

### Beispiel 10

Luquafleece IS wurde eine Minute bei 150 °C und 10 bar komprimiert. Es wurden 30 x 50 mm Proben vermessen. Dabei wurden folgende Daten in 0,9 %iger NaCl erhalten:
Dicke des komprimierten Materials: 0,4 mm
CRC: 6,9 g / g
CRC: 2128 g / m²
Teebeutelwert: 10,5 g / ccm*)
Retention: 4,3 g / ccm*)
Dichte des eingesetzten Materials: 0,590 g / ccm
Ausdehnungsfaktor in z-Achse: 11,2
AAP (0,7 psi): 12,3 g / g
AAP (0,7 psi): 3251 g / m²
AAP (0,7 psi): 6,5 g / ccm
*) die Angabe bezieht sich auf das eingesetzte Material

### Bespiel 11

Luquafleece IS wurde eine Minute bei 200 °C und 160 bar komprimiert. Es wurden 30 x 50 mm Proben vermessen. Dabei wurden folgende Daten in 0,9 %iger NaCl erhalten:
Dicke des komprimierten Materials: 0,22 mm
CRC: 8,2 g / g
CRC: 2230 g / m²
Teebeutelwert: 15,5 g / ccm*)
Retention: 7,4 g / ccm*)
Dichte des eingesetzten Materials: 1,043 g / ccm
Ausdehnungsfaktor in z-Achse: 18,9
AAP (0,7 psi): 11,8 g / g
AAP (0,7 psi): 3251 g / m²
AAP (0,7 psi): 10,9 g / ccm
*) die Angabe bezieht sich auf das eingesetzte Material
Vergleichsbeispiel 4 (Luquafleece IS)
Dicke des Luquafleece IS: 5,2 mm
CRC: 5,8 g / g
CRC: 1495 g / m²
Teebeutelwert: 0,8 g / ccm*)
Retention: 0,3 g / ccm*)
Dichte des eingesetzten Materials: 0,047 g / ccm
Ausdehnungsfaktor in z-Achse: 1,1
AAP (0,7 psi): 13,0 g / g
AAP (0,7 psi): 3300 g / m²
AAP (0,7 psi): 0,6 g / ccm
*) die Angabe bezieht sich auf das eingesetzte Material
Vergleichsbeispiel 5 (Luquafleece IS 48 s bei 6 bar und 50 °C komprimiert)
Dicke des komprimierten Materials: 3,1 mm
CRC: 5,9 g / g
CRC: 1346 g / m²
Teebeutelwert: 1,3 g / ccm*)
Retention: 0,4 g / ccm*)
Dichte des eingesetzten Materials: 0,072 g / ccm
Ausdehnungsfaktor in z-Achse: 1,7
AAP (0,7 psi): 12,3 g / g
AAP (0,7 psi): 2968 g / m²
AAP (0,7 psi): 1,0 g / ccm
*) die Angabe bezieht sich auf das eingesetzte Material

Ein Erhöhen des Druckes bei niedrigen Temperaturen ergibt keine signifikante Verbesserung. Dies soll anhand des folgenden Beispiels belegt werden.
Vergleichsbeispiel 6 (Luquafleece IS 60 s bei 80 bar und 50 °C komprimiert)
Dicke des komprimierten Materials: 0,8 mm
Dicke des komprimierten Materials nach 4 Wochen: 1,2 mm
CRC: 5,8 g / g
CRC: 1330 g / m²
Teebeutelwert: 1,0 g / ccm*)
Retention: 0,3 g / ccm*)
Dichte des eingesetzten Materials: 0,184 g / ccm
Ausdehnungsfaktor in z-Achse: 2,8
AAP (0,7 psi): 11,7 g / g
AAP (0,7 psi): 2686 g / m²
AAP (0,7 psi): 3,0 g / ccm
*) die Angabe bezieht sich auf das eingesetzte Material

### Beeinflussung der Härte des ultradünnen Materials

Härte und Feuchtigkeitsaufnahmegeschwindigkeit lassen sich z.B. durch Zugabe von weichmachenden Chemikalien oder durch eine Strukturierung (Vergrößerung) der Oberfläche des ultradünnen Materials beeinflussen. Als weichmachende Chemikalien kommen z.B. tertiäre Alkanolamine in Frage. Die freien Säuregruppen des SAP werden dabei bevorzugt zumindest zu 20 mol% neutralisiert. Bevorzugte Alkanolamine sind ausgewählt aus der Gruppe Triethanolamin, Methyldiethanolamin, Dimethylaminodiglycol, Dimethylethanolamin, N,N,N',N'-Tetra(hydroxyethyl)ethylendiamin. Diese Möglichkeiten sind an sich nicht neu, wohl aber im Zusammenhang mit einer Beeinflussung des Wasseraufnahmevermögens bei superabsorbierenden Flächengebilden. Daneben lässt sich das ultradünne Material durch eine gezielte Zugabe geringer Mengen Wasser weicher machen. Die angesprochenen Möglichkeiten des Weichmachens können auch kombiniert werden. Um eine möglichst homogene Erhöhung der Feuchtigkeit zu erreichen, wird das Material vorzugsweise mit Wasserdampf oder Wassernebel behandelt. Um die Weichheit zu bewahren ist es allerdings anschließend erforderlich, das Material luftdicht zu verpacken.

Die Veränderung der Härte kann beispielsweise mit einer Apparatur gemessen werden, mit der eine Kugel eine definierte Wegstrecke in das Material gedrückt und die Kraft gemessen wird, die für diese Wegstrecke erforderlich ist. Das ultradünne Material wurde 24 h bei Raumtemperatur in eine gesättigte Wasserdampfatmosphäre gelegt. Das Material nahm in diesem Zeitraum 70 % seines Eigengewichts an Feuchtigkeit auf. Nach 24 h wurde die Probe aus dieser Atmosphäre entnommen und es wurde die Kraft gemessen, die erforderlich ist, die Kugel 10 mm tief in das Material hineinzudrücken. Im Stundenrhythmus wurde die Messung wiederholt. Das Material befand sich in dieser Zeit in einer Umgebung mit 50 % rel. Feuchte bei 24 °C, d.h. der Feuchtigkeitsgehalt nahm in diesem Zeitraum stetig ab und hatte nach 7 h den Ausgangswert nahezu wieder erreicht.

Folgende Feuchtigkeitsgehalte (bezogen auf das eingesetzte Material vor Feuchtlagerung) wurden gemessen:
70 % nach 24 h in gesättigter Wasserdampfatmosphäre
40 % nach 1 h bei 24 °C und 50 % rel. Luftfeuchte
11 % nach 3 h bei 24 °C und 50 % rel. Luftfeuchte
1 % nach 7 h bei 24 °C und 50 % rel. Luftfeuchte

Die für die Härtemessung eingesetzten Materialien waren 1 Minute bei 160 bar und unterschiedlichen Temperaturen komprimiert worden. Folgende Meßwerte (10 mm Weglänge) wurden erhalten:
Probe 1 (200 °C / 160 bar):
   Kraft: 8 N Messung sofort, 10 N nach 1 h, 21 N nach 3 h, 33 N nach 7 h
Probe 2 (150 °C / 160 bar):
   Kraft: 7 N Messung sofort, 9 N nach 1 h, 18 N nach 3 h, 24 N nach 7 h
Probe 3 (50 °C / 160 bar):
   Kraft: 8 N Messung sofort, 11 N nach 1 h, 16 N nach 3 h, 19 N nach 7 h
   Vergleich: Luquafleece IS
Kraft: 4 N Messung sofort, 4,5 N nach 1 h, 6 N nach 3 h, 10 N nach 7 h

Es ist deutlich zu erkennen, dass die Materialien mit abnehmendem Feuchtigkeitsgehalt härter werden. Weiterhin ist den Meßwerten zu entnehmen, dass in etwa die gleiche Kraft aufgewendet werden muß, die Kugel 10 mm in ein trockenes Luquafleece hineinzudrücken wie in ein ultradünnes Material mit einem Feuchtegehalt von 70 %.

## Patentansprüche

1. Material aus superabsorbierendem Polymer und Fasern, erhältlich durch in-situ Aufpolymerisation des superabsorbierenden Polymeren auf die Fasern und Pressen bei einer Temperatur von mindestens 60° C und höchstens 200 °C bei einem Druck von mindestens 3 bar.

2. Materialien gemäß Anspruch 1 erhältlich durch Pressen bei einer Temperatur von mindestens 70° C.

3. Materialien gemäß Anspruch 1 erhältlich durch Pressen bei einer Temperatur von mindestens 80° C.

4. Materialien nach einem der Ansprüche 1 bis 3, erhältlich durch Pressen bei einem Druck von mindestens 5 bar.

5. Materialien nach einem der Ansprüche 1 bis 3, erhältlich durch Pressen bei einem Druck von mindestens 10 bar.

6. Material gemäß einem der Ansprüche 1 bis 5, das sich bei Wasserzugabe in einer Dimension um das mindestens das 5-fache ausdehnt und in den zwei anderen Dimensionen um weniger wie 20%.

7. Material gemäß einem der Ansprüche 1 bis 6, das sich bei Wasserzugabe in einer Dimension um das mindestens das 10-fache ausdehnt und in den zwei anderen Dimensionen um weniger wie 10%.

8. Material gemäß einem der Ansprüche 1 bis 7, mit eine Dichte von mindestens 0.5 g/ccm bis zu einer Dichte von 1.2 g/ccm.

9. Material gemäß einem der Ansprüche 1 bis 8, bei dem das Verhältnis von Teebeutel zu Retention in 0.9%iger NaCl-Lösung größer 2 ist.

10. Material gemäß einem der Ansprüche 1 bis 9, bei dem die Retention in 0.9%iger NaCl-Lösung größer 3 g/ccm ist.

11. Material gemäß einem der Ansprüche 1 bis 10, bei dem die Zunahme der Dicke nach 60 Tagen nach Komprimierung weniger wie 100% bezogen auf die Dicke direkt nach der Komprimierung beträgt.

12. Material gemäß einem der Ansprüche 1 bis 11, bei dem die FSEV (Ausdehnungsvolum) nach 60 Sekunden im Vergleich zum unkomprimierten Material mindestens verdoppelt ist.

13. Material gemäß einem der Ansprüche 1 bis 12, bei dem die FSEV nach 2 Minuten im Vergleich zum unkomprimierten Material mindestens 60% höher ist.

14. Material gemäß einem der Ansprüche 1 bis 13, bei dem die EVUL (Ausdehnungsvolum unter einer Duckeinwirkung) nach 60 Sekunden im Vergleich zum unkomprimierten Material mindestens verdoppelt ist.

15. Material gemäß einem der Ansprüche 1 bis 14, bei dem die EVUL nach 2 Minuten im Vergleich zum unkomprimierten Material mindestens 60% höher ist.

16. Material gemäß einem der Ansprüche 1 bis 15, bei dem die AAP (Ausdehnungstaktor) (0.7psi) in 0.9%iger NaCl-Lösung größer 5 g/ccm ist.

17. Mehrschichtmaterialien enthaltend Material nach einem der Ansprüche 1 bis 16.

18. Verwendung von Material und Mehrschichtmaterial gemäß einem der Ansprüche 1 bis 17 zur Absorption von Wasserdampf.

19. Verwendung von Material und Mehrschichtmaterial gemäß einem der Ansprüche 1 bis 17 zur Absorption von Wasser oder wässrigen Flüssigkeit, insbesondere Körperflüssigkeit.

20. Verfahren zur Herstellung von komprimierten Material enthaltend superabsorbierendes Polymer und Fasern, erhältlich durch in-situ Aufpolymerization des superabsorbierenden Polymers auf die Fasern und Pressen bei einer Temperatur von mindestens 60 °C und höchstens 200 °C bei einem Druck von mindestens 3 bar.

## Claims

1. Material comprising superabsorbent polymer and fibers, obtainable by in situ polymerization of the superabsorbent polymer onto the fibers and pressing at not less than 3 bar at not less than 60°C and not more than 200°C.

2. Materials according to claim 1 that are obtainable by pressing at not less than 70°C.

3. Materials according to claim 1 that are obtainable by pressing at not less than 80°C.

4. Materials according to any of claims 1 to 3 that are obtainable by pressing at not less than 5 bar.

5. Materials according to any of claims 1 to 3 that are obtainable by pressing at not less than 10 bar.

6. Material according to any of claims 1 to 5 that expands not less than 5-fold in one dimension and by less than 20% in the other two dimensions on addition of water.

7. Material according to any of claims 1 to 6 that expands not less than 10-ford in one dimension and by less than 10% in the other two dimensions on addition of water.

8. Material according to any of claims 1 to 7 that has a density in the range from not less than 0.5 g/ccm to 1.2 g/ccm.

9. Material according to any of claims 1 to 8 where the ratio of teabag to retention in 0.9% NaCl solution is greater than 2.

10. Material according to any of claims 1 to 9 where the retention in 0.9% NaCl solution is greater than 3 g/ccm.

11. Material according to any of claims 1 to 10 where the increase in thickness 60 days after compression is less than 100% based on the thickness directly after compression.

12. Material according to any of claims 1 to 11 where the FSEV (free swell expansion volume) after 60 seconds is at least double that of the uncompressed material.

13. Material according to any of claims 1 to 12 where the FSEV after 2 minutes is at least 60% higher than that of the uncompressed material.

14. Material according to any of claims 1 to 13 where the EVUL (expansion volume sunder load) after 60 seconds is at least double that of the uncompressed material.

15. Material according to any of claims 1 to 14 where the EVUL after 2 minutes is at least 60% higher than that of the uncompressed material.

16. Material according to any of claims 1 to 15 where the AAP (absorption against pressure) (0.7 psi) in 0.9% NaCl solution is greater than 5 g/ccm.

17. Laminates comprising material according to any of claims 1 to 16.

18. The use of material and laminate according to any of claims 1 to 17 to absorb water vapor.

19. The use of material and laminate according to any of claims 1 to 17 to absorb water or aqueous fluid, especially body fluid.

20. The process for producing compressed material comprising superabsorbent polymer and fibers, obtainable by in situ polymerization of the superabsorbent polymer onto the fibers and pressing at not less than 3 bar at not less than 60°C and not more than 200°C.

## Revendications

1. Matériau à base de polymère superabsorbant et de fibres, pouvant être obtenu par polymérisation in situ du polymère superabsorbant sur les fibres et compression à une température supérieure ou égale à 60 °C et inférieure ou égale à 200 °C à une pression supérieure ou égale à 3 bars.

2. Matériaux selon la revendication 1, pouvant être obtenus par compression à une température supérieure ou égale à 70 °C.

3. Matériaux selon la revendication 1, pouvant être obtenus par compression à une température supérieure ou égale à 80 °C.

4. Matériaux selon l'une quelconque des revendications 1 à 3, pouvant être obtenus par compression à une pression supérieure ou égale à 5 bars.

5. Matériaux selon l'une quelconque des revendications 1 à 3, pouvant être obtenus par compression à une pression supérieure ou égale à 10 bars.

6. Matériau selon l'une quelconque des revendications 1 à 5, qui, par ajout d'eau, se dilate au moins d'un facteur de 5 dans une dimension et de moins de 20 % dans les deux autres dimensions.

7. Matériau selon l'une quelconque des revendications 1 à 6, qui, par ajout d'eau, se dilate au moins d'un facteur de 10 dans une dimension et de moins de 10 % dans les deux autres dimensions.

8. Matériau selon l'une quelconque des revendications 1 à 7, ayant une densité supérieure ou égale à 0,5 g/cm³ jusqu'à une densité de 1,2 g/cm³.

9. Matériau selon l'une quelconque des revendications 1 à 8, dans lequel le rapport d'un sachet de thé à l'égard de la rétention dans une solution de NaCl à 0,9 % est supérieur à 2.

10. Matériau selon l'une quelconque des revendications 1 à 9, dans lequel la rétention dans une solution de NaCl à 0,9 % est supérieure à 3 g/cm³.

11. Matériau selon l'une quelconque des revendications 1 à 10, dans lequel l'augmentation de l'épaisseur après 60 jours après compression est inférieure à 100 % par rapport à l'épaisseur directement après la compression.

12. Matériau selon l'une quelconque des revendications 1 à 11, dans lequel le FSEV (volume de dilatation) après 60 secondes est au moins doublé en comparaison du matériau non comprimé.

13. Matériau selon l'une quelconque des revendications 1 à 12, dans lequel le FSEV après 2 minutes est au moins 60 % supérieur en comparaison du matériau non comprimé.

14. Matériau selon l'une quelconque des revendications 1 à 13, dans lequel l'EVUL (volume de dilatation sous l'effet d'une pression) après 60 secondes est au moins doublé en comparaison du matériau non comprimé.

15. Matériau selon l'une quelconque des revendications 1 à 14, dans lequel l'EVUL après 2 minutes est au moins 60 % supérieur en comparaison du matériau non comprimé.

16. Matériau selon l'une quelconque des revendications 1 à 15, dans lequel l'AAP (absorption sous l'effet d'une pression) (0,7 psi) dans une solution de NaCl à 0,9 % est supérieur à 5 g/cm³.

17. Matériaux multicouches contenant le matériau selon l'une quelconque des revendications 1 à 16.

18. Utilisation du matériau et du matériau multicouche selon l'une quelconque des revendications 1 à 17 pour l'absorption de la vapeur d'eau.

19. Utilisation du matériau et du matériau multicouche selon l'une quelconque des revendications 1 à 17 pour l'absorption de l'eau ou d'un liquide aqueux, notamment un liquide corporel.

20. Procédé de fabrication d'un matériau comprimé contenant un polymère superabsorbant et des fibres, pouvant être obtenu par polymérisation in situ du polymère superabsorbant sur les fibres et compression à une température supérieure ou égale à 60 °C et inférieure ou égale à 200 °C à une pression supérieure ou égale à 3 bars.
